# EUROPÄISCHE PATENTSCHRIFT

(11) **EP 2 475 979 B1**
(45) Veröffentlichungstag und Bekanntmachung des Hinweises auf die Patenterteilung: **11.12.2013**
(21) Anmeldenummer: 10760912.5
(22) Anmeldetag: 09.09.2010
(51) Int. Cl.: G01N 29/036, G01N 33/86

(54) **VERFAHREN ZUR BESTIMMUNG DER THROMBOZYTENFUNKTION MIT EINEM RESONATOR**
METHOD FOR DETERMINING THROMBOCYTE FUNCTIONS BY MEANS OF A RESONATOR
PROCÉDÉ DE DÉTERMINATION DE LA FONCTION DE THROMBOCYTES AU MOYEN D'UN RÉSONATEUR

(30) Priorität: 09.09.2009 DE 102009040881
(43) Veröffentlichungstag der Anmeldung: 18.07.2012
(73) Patentinhaber: Andreas Hettich GmbH & Co. KG, 78532 Tuttlingen (DE)
(72) Erfinder: GEHRING, Frank, K., 72364 Obernheim (DE); WENDEL, Hans-Peter, 72336 Balingen (DE); SINN, Stefan, 71083 Herrenberg (DE); MÜLLER, Lothar, 65205 Wiesbaden (DE)
(74) Vertreter: Borchert, Uwe Rudolf
(86) Internationale Anmeldenummer: PCT/EP2010/005548
(87) Internationale Veröffentlichungsnummer: WO 2011/029600

(56) Entgegenhaltungen:
- TAKEHISA MATSUDA ET AL: "NOVEL INSTRUMENTATION MONITORING IN SITU PLATELET ADHESIVITY WITH A QUARTZ CRYSTAL MICROBALANCE", ASAIO JOURNAL, LIPPINCOTT WILLIAMS & WILKINS / ASAIO, HAGERSTOWN, MD, US, Bd. 38, Nr. 3, 1. Juli 1992 (1992-07-01), Seiten 171-173, XP000321538, ISSN: 1058-2916

## Beschreibung

Die Erfindung betrifft ein Verfahren zur Bestimmung der Thrombozytenfunktion gemäß der im Oberbegriff des Anspruchs 1 angegebenen Art.

Für die Blutgerinnung müssen sowohl die primäre als auch die sekundäre Hämostase funktionsfähig sein. Zur Überprüfung der sekundären Hämostase sind viele Möglichkeiten bekannt. Für die primäre Hämostase, die sich in Adhäsion und Aggregation von Thrombozyten unterteilt, ist dem Stand der Technik kein ausreichend genaues Analyseverfahren zur Bestimmung der spezifischen Thrombozyten-funktion zu entnehmen.

Nach dem Stand der Technik sind Blutanalysen, welche die Hämostase betreffen möglich. Jedoch ist eine Unterscheidung, bei der primären Hämostase, ob Adhäsion oder Aggregation geschädigt ist, oder in welcher Art die Aggregation gehemmt ist, nicht möglich.

Es ist bekannt, dass Blutplättchen auf der Oberfläche eines Schwingquarzes durch Adhäsion angelagert werden können. Diese Anlagerung ist qualitativ messbar, lässt jedoch keine spezifische Identifikation einer Fehlfunktion der Aggregation zu.

Die Veröffentlichung "real-time monitoring of adhesion and aggregation of platelets using thickness shear mode sensors" (E.Ergezen et al., Biosensors und Bioelectronics 23 (2007), 575-582) beschreibt eine Analyse der Blutgerinnungsphasen von Adhäsion und Aggregation von Blutplättchen mittels eines Schwingquarzes. Diese Schrift zeigt, dass verschiedene Phasen, Adhäsion, und Aggregation mit Hilfe eines Schwingquarzes qualitativ nachweisbar ist. Sie löst nicht das Problem, die Funktionsfähigkeit der Thrombozyten zu Adhäsion und Aggregation zu unterscheiden. Aus dieser Druckschrift ist bekannt, dass die Aggregation von mangelnder Adhäsionsfähigkeit gut abgrenzbar ist, da eine erfolgreiche Aggregation eine hohe Frequenz bzw. Dämpfungsänderung nach sich zieht. Entsprechend ist bekannt die Aggregationsfähigkeit festzustellen. Zwischen Adhäsionsfähigkeit und Aggregationsfähigkeit der Thrombozyten kann nach diesem Verfahren nicht zuverlässig unterschieden werden, da der Frequenzhub nicht nur von der Zugabe von ADP abhängig ist sondern auch von der Thrombozytenkonzentration, der Bindungsstärke der Schwingquarzoberfläche und der Adhäsionsfähigkeit der Thrombozyten. Durch diese Betrachtung kann möglicherweise eine falschpositive Entscheidung erfolgen, da lediglich zwischen einem erfolgten Frequenzabfall und keinem Frequenzabfall unterschieden wird, wobei auch lediglich die Adhäsion der Thrombozyten unter bestimmten Vorraussetzungen zu einem starken Frequenzabfall führen kann. Auch die US 2005/0015001 A1 beschreibt im Wesentlichen das oben genannte.

Die Druckschrift "A new method for continuous measurement of platelet adhesion under flow conditions" (K. Kawakami et al., ASAIO Journal 39(1993), M558-M560) offenbart, dass eine Adhäsion von Plättchen messbar ist, stellt aber keinen Bezug zur Analyse einer Dysfunktion noch einer Kombination mit einer Bewertung der Aggregationsfunktion her.

Im Vortrag "Potenzial der Schwingquarzsensoren zur inline Hämothase-Prüfung" (F.K.Gehring, Miniaturisierte Biosystemtechnik, BMBF, Hannover, 7.10.2008) auf Folie 24 sind zwei Frequenzverläufe dargestellt, die Rückschluss auf die Thrombozytenfunktion gelben sollen. Eine Darstellung zeigt einen Frequenzabfall bei einer Schwingquarzmessung von aktiviertem Blut, die zweite Kurve zeigt keinen Frequenzabfall bei inhibiertem Blut. Dies gibt in der Form lediglich wieder, dass sich eine Funktion anhand des Verhaltens der Schwingungsparameter ablesen lässt. Es ist aufgrund dieser Unterlagen unklar, ob die dargelegte Funktion eine Adhäsion, Aggregation, oder sonstige Ablagerung eines Stoffs auf der Schwingquarzoberfläche abbildet. Eine eindeutige Funktionszuordnung kann dieser Offenbarung nicht entnommen werden.

Auch die Druckschrift "Novel instrumentation monitoring in situ platelet adhesivity with a quartz crystal microbalance" (T. Matsuda, ASAIO Journal 38 (1992), M171-M173) beschreibt eine Untersuchung der Thrombozytenfunktion mittels eines Schwingquarzes, wobei der zeitliche Verlauf der Resonanzfrequenz beobachtet wird.

Es ist die Aufgabe der Erfindung ein Verfahren anzugeben mittels dem eine Aussage getroffen werden kann, welcher Zweig der zellulär vermittelten Gerinnung gestört ist

Die Aufgabe wird durch die kennzeichnenden Merkmale des Anspruchs 1 gelöst.

In bekannter Weise werden zur Bestimmung von Parametern der primären Hämostase die Schwingungsparameter eines Resonators gemessen, der mit einer Oberfläche versehen ist, die in Kontakt mit einem Thrombozyten enthaltenden Probenfluid steht Dadurch lagern sich Thrombozyten oder andere Blutbestandteile an der Oberfläche des Schwingquarzes an, die dessen Schwingungsverhalten beeinflussen.

Erfindungsgemäß wird zur Bestimmung der Thrombozytenfunktion ein Probenfluid auf eine Resonatoroberfläche und eine Analyse der Charakteristik beziehungsweise Kinetik der oder des gemessenen Schwingungsparameters durchgeführt, aufgrund der die Thrombozytenfunktion bewertet wird. Anhand der Charakteristik wird zwischen totaler Dysfunktion, bzw. Adhäsionsstörung und Aggregationsstörung der Thrombozyten unterschieden. Es wird die Charakteristik des Verlaufs der Schwingungsparameter über die Zeit ausgewertet und abhängig davon wird eine Aussage getroffen, welcher Zweig der zellulär vermittelten Gerinnung gestört ist. Durch dieses Verfahren wird eine Diagnostik mit einem Resonator ermöglicht.

Dies hat den enormen Vorteil, dass durch eine Unterscheidung welcher Zweig der Gerinnung gestört ist eine entsprechend abgestimmte Medikation erfolgen kann. Grundsätzlich wird aufgrund dieser Untersuchungsmöglichkeiten eine großer Informationspool der zellulär vermittelten Gerinnung bereitgestellt. Eine gezielte Medikation vermindert die Nebenwirkungen für den Patienten deutlich und können bedeutend Kosten sparen.

Erfindungsgemäß werden die Schwingungsparameter in Form der Resonanzfrequenz ausgewertet. Insbesondere wird die Grundschwingung betrachtet.

Nach Starten der Messung wird ein Thrombozyten enthaltendes Probefluid auf den Schwingquarz aufgebracht Verändert sich die Kurve der Schwingungsparameter über die Zeit nicht, wird auf eine Dysfunktion in der Thrombozytenfunktion geschlossen. Dies bedeutet, dass die Thrombozyten nicht adhärieren können, was zur Folge hat, dass auch keine Aggregation möglich ist. Eine weitere Untersuchung ist nicht notwendig, da sofern keine Adhäsion möglich ist auch keine Blutgerinnung erfolgt. Die Dysfunktion entspricht also einer Adhäsionsstörung.

Im Gegensatz dazu schließt man bei einem mehr oder weniger linearen Abfall der Schwingungsparameter über die Zeit auf eine grundsätzliche Adhäsionsfähigkeit der Thrombozyten. Die Adhäsion kann durch Zusatz eines Inhibitors verhindert werden. So kann beispielsweise der GPIIb/IIIa - Rezeptor blockiert werden, um eine Adhäsion zu vermeiden. Es ist also möglich, dass nachdem eine Probe positiv auf ihre Adhäsionsfunktion getestet wurde der Versuch mit künstlich gehemmten Thrombozytenfunktion wiederholt wird, um das Ergebnis zu verifizieren.

Nach dem Verfahren werden die Thrombozyten in einem nicht aktivierten Probenfluid, das auf einen Schwingquarz aufgebracht wird aufgrund des Abfalls von Schwingungsparametern als adhäsionsfähig eingestuft.

Um die Aggregationsfähigkeit der Thrombozyten im Probenfluid zu untersuchen, muss dieses aktiviert werden. Die Aktivierung der Probe erfolgt durch Zugabe eines Aktivators. Der Aktivator kann entweder kurz vor der Applikation der Probe eingebracht werden oder der Schwingquarz selbst weist an seiner Oberfläche einen bereits eingebrachten Aktivator auf. Entsprechend dem Verfahren wird auch hier der Schwingungsparameter über die Zeit aufgezeichnet. Stellt man einen sehr starken Abfall der Schwingungsparameter etwa in exponentieller Form fest, werden die Thrombozyten als aggregationsfähig eingestuft.

Entspricht das Schwingungsverhalten des Schwingquarzes mit aktivierter Probe dem der nicht aktivierten Probe, so wird darauf geschlossen, dass die Thrombozyten adhäsionsfähig aber nicht aggregationsfähig sind. Es wird auf eine Aggregationsstörung geschlossen.

Darüber hinaus kann durch Zugabe verschiedener Aktivatoren ermittelt werden, welcher Rezeptor der Thrombozyten eine Dysfunktion aufweist. Die unterschiedlichen Aktivierungszweige können somit über Zugabe von entweder van Willebrand Faktor (vWF), Arachidonsäure (AA), Adenosin-Diphosphat (ADP) oder Fibrinogen ausgelöst werden, was eine Eingrenzung der Dysfunktion der entsprechenden Aktivierungszweige erlaubt

Diese Eingrenzung kann auch dadurch erfolgen, dass bei einer erfolgreichen Aggregation der Thrombozyten unterschiedliche Rezeptoren blockiert werden. Sind alle Rezeptoren bis auf einen zu untersuchenden Rezeptor blockiert, kann festgestellt werden, ob dieser Rezeptor eine Störung aufweist, indem das Aggregationsverhalten der aktivierten Probe gemessen wird und man bei einem Verlauf der Schwingungsparameter, der einer Adhäsion entspricht, auf eine Störung des nicht blockierten Rezeptors schließen kann.

Insbesondere durch Aktivierung und Blockierung einzelner Rezeptoren, werden jene identifiziert welche Funktionsstörungen aufweisen. Des weiteren ist die Blockierung oder Aktivierung einzelner Gruppen von Rezeptoren durch einen spezifischen Antikörper oder Teile davon vorgesehen. Darüber hinaus kann diese Funktion auch durch andere spezifisch bindende Moleküle, wie zum Beispiel Lektine oder Fragmente von Nukleinsäuren bewirkt werden.

Vorteilhafte Auswirkungen hat es auch, einen Aktivator, wie beispielsweise Fibrinogen bereits in die Oberfläche beispielsweise eines Schwingquarzes zu integrieren. Man umgeht dabei das Problem, dass der Zeitpunkt von der Aktivierung der Probe bis zur Messung nicht besonders kurz sein muss und daher die gesamte Vorrichtung einfacher gestaltet werden kann. Die Thrombozyten adhärieren an der Fibrinogenschicht und werden gleichzeitig aktiviert, wodurch die Aggregation ausgelöst wird.

Weitere Vorteile, Merkmale und Anwendungsmöglichkeiten der vorliegenden Erfindung ergeben sich aus der nachfolgenden Beschreibung in Verbindung mit den in der Zeichnung dargestellten Ausführungsbeispielen. Die Erfindung wird im Folgenden anhand der Zeichnung näher beschrieben.

In der Zeichnung bedeuten:
- Fig. 1: einen Verfahrensablauf zur Bestimmung der Thrombozytenfunktion, und
- Fig. 2: schematische Darstellung des Verlaufs von Schwingungsparametern für Dysfunktion und Adhäsion und Aggregation.

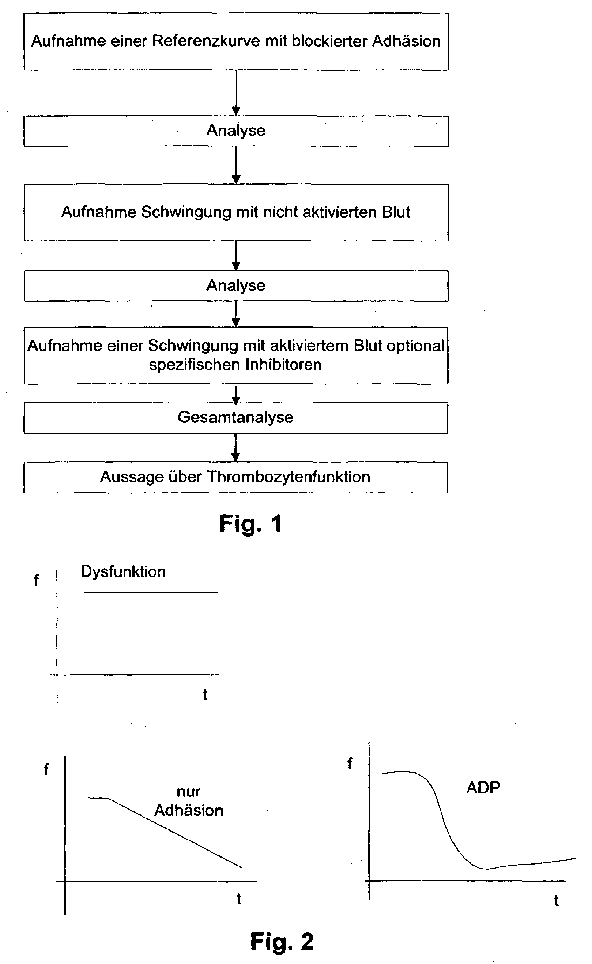

In Fig.1 ist der Ablauf zur Untersuchung der Thrombozytenfunktion einer Blutprobe dargestellt. Als erstes werden Thrombozyten, die nicht aktiviert sind und deren Adhäsionsfähigkeit blockiert ist auf einen Schwingquarz aufgebracht. Die Adhäsionsfähigkeit kann künstlich gestört werden, indem beispielsweise der Collagenrezeptor oder GPIb-IX Rezeptor durch Abciximab blockiert wird. Dies verhindert die Adhäsion der Thrombozyten auf der Schwingquarzoberfläche, wodurch eine unbelastete Referenzkurve der Resonanzfrequenz aufgenommen wird.

Nach Aufnahme der Referenzkurve wird eine weiteres Probenfluid entweder auf den selben Schwingquarz oder auf den gereinigten Schwingquarz aufgebracht. Dieses Probenfluid enthält Thrombozyten deren Rezeptoren bezüglich der Adhäsion weder blockiert noch aktiviert sind. Anschließend erfolgt die Analyse der Kurve. Fällt die Resonanzfrequenz über die Zeit stärker ab, als dies bei der Probe mit den gehemmten Adhäsionsrezeptoren der Fall war, sind die Thrombozyten in dem Probenfluid adhäsionsfähig, und es schließt sich ein weiterer Messschritt an.

In einem weiteren Schritt wird eine mit einem Aktivator induziertes Probenfluid beispielsweise mit Adenosin-Diphosphat (ADP), van Willebrand Faktor (vWF) oder einem anderen Faktor auf die Schwingquarzoberfläche aufgebracht. Fällt die gemessene Resonanzfrequenz über die Zeit stärker ab, als im vorangegangenen Schritt, in dem die Adhäsion gemessen wurde, lässt sich dann bei einer Aktivierung durch Fibrinogen darauf schließen, dass die primäre Aggregation funktionsfähig ist. In einem weiteren Schritt wird ein Aktivator oder eine mit einem Aktivator induzierte Probe zugegeben, was die sekundäre Aggregation auslöst.

Nimmt die Resonanzfrequenz über die Zeit deutlich stärker ab, als zuvor, ist auch die sekundäre Aggregation funktionsfähig. Beeinflussen entsprechend zugegebene Aktivatoren das Schwingungsverhalten des Resonators nicht, wird auf eine Fehlfunktion der korrespondierenden Rezeptoren geschlossen.

Durch diesen Ablauf ist ein grundsätzlicher Ablauf gegeben, der darin besteht zuerst eine inhibierte, dann eine nicht-aktivierte und anschließend eine aktivierte Probe, unterbrochen durch beliebige Spülschritte, zu vermessen. Darüber hinaus können auf diese Weise mehrere Kombinationen aus Inhibition und Aktivierung in diesen Ablauf aufgenommen werden.

Auf diese Weise lässt sich eindeutig der gestörte Zweig der zellulär vermittelten Gerinnung ermitteln.

Fig. 2 zeigt drei Frequenzverläufe des Schwingquarzes über die Zeit von drei unterschiedlichen Throm-bozytenfunktionen. Der erste Frequenzverlauf zeigte eine gerade Kurve über die gesamte Zeit. Ein Frequenzabfall ist in diesem Fall nicht festzustellen. Dies bedeutet, dass sich keine oder vernachlässigbar wenige Thrombozyten an der Schwingquarzoberfläche anlagern. Ein solcher Frequenzverlauf lässt eindeutig auf eine Dysfunktion der Thrombozyten schließen. Möglicherweise ist dieser auch durch eine bewusste Blockade durch Zugabe von Abciximab daran gehindert sich an der Schwingquarzoberfläche anzulagern. Die Thrombozyten sind nicht in der Lage zu adhärieren, entweder aufgrund einer Dysfunktion oder weil diese beispielsweise mit Abciximab bewusst gehemmt wurden.

Die zweite Kurve zeigt einen linear abfallenden Verlauf der Frequenz über die Zeit. Dies zeigt, dass sich nach und nach eine zunehmende Anzahl von Thrombozyten und andere Blutbestandteile an der Schwingquarzoberfläche anlagern. Ein derartiger Frequenzverlauf lässt den eindeutigen Schluss zu, dass eine Adhäsion stattfindet.

In einem dritten Graph ist eine stark abfallende Frequenzkurve dargestellt. Ein solcher Kurvenverlauf kann lässt auf das Vorhandensein funktionsfähiger Thrombozyten schließen, die mindestens einen funktionsfähigen Rezeptor für die Aggregation aufweisen. Für eine Aggregation muss das Blut aktiviert werden. Dies kann mittels Adenosin-Diphosphat (ADP) Fibrinogen oder dem van Willebrand Faktor (vWF) ausgelöst werden. Möglich ist auch eine Aktivierung mittels Arachidonsäure (AA). Abhängig vom verwendeten Aktivator beziehungsweise einem etwaig angewandten selektiven Inhibitor kann so die Funktionsfähigkeit der einzelnen Rezeptoren der Thrombozyten überprüft werden.

## Patentansprüche

1. Verfahren zur Bestimmung von Parametern der Hämostase mittels der Messung von Schwingungsparametern eines Resonators mit einer Messoberfläche, die in Kontakt mit einem Thrombozyten enthaltenden Probenfluid steht, wobei als Schwingungsparameter die Resonanzfrequenz ausgewertet wird **gekennzeichnet durch** die Analyse der Charakteristik eines Schwingungsparameters über die Zeit, aufgrund welcher die Thrombozytenfunktion bewertet wird und zwischen Adhäsionsstörung und Aggregationsstörung unterschieden wird, wobei das Probefluid aktiviert wird und bei einer im Wesentlichen exponentiellen Abfall der Schwingungsparameter, die Thrombozyten als funktionsfähig eingestuft werden, bei einer im Wesentlichen linearen Abfall der Schwingungsparameter, die Thrombozytenfunktion als Aggregations-Dysfunktion jedoch mit einer funktionierenden Adhäsions-Funktion eingestuft wird, und bei unveränderten Schwingungsparametern auf eine Adhäsions-Dysfunktion geschlossen wird.

2. Verfahren nach Anspruch 1, **dadurch gekennzeichnet, dass** die Thrombozytenfunktion durch Zugabe verschiedener Aktivatoren, die unterschiedliche Aktivierungszweige auslösen, näher spezifiziert wird.

3. Verfahren nach einem der vorangegangenen Ansprüche, **dadurch gekennzeichnet, dass** das Aktivierungszweige durch Zugabe entsprechender Inhibitoren blockiert werden.

4. Verfahren nach den Ansprüchen 2 oder 3, **dadurch gekennzeichnet, dass** durch Aktivierung und Blockierung einzelner Rezeptoren diejenigen identifiziert werden, die Funktionsstörungen aufweisen.

5. Verfahren nach einem der vorangegangenen Ansprüche, **dadurch gekennzeichnet, dass** die Blockierung oder Aktivierung einzelner oder Gruppen von Rezeptoren durch einen spezifischen Antikörper, durch Teile davon, oder durch andere spezifisch bindende Moleküle wie Lektine, Fragmente von Nukleinsäuren oder Ähnlichem bewirkt wird.

## Claims

1. A method for determining haemostasis parameters by measuring vibration parameters of a resonator having a measuring surface which contacts a platelet-containing sample fluid, in which the resonance frequency is used as the vibration parameter to be analysed, **characterized by** the analysis of the characteristic of a vibration parameter over time, based on which the platelet function will then be assessed and a distinction will be made between the presence of an adhesion disorder or that of an aggregation disorder, in which the sample fluid will be activated and the platelets will be considered functional if the drop of the vibration parameters is more or less exponential, the platelet function will be considered dysfunctional as regards aggregation but functional as regards adhesion if the drop of the vibration parameters is more or less linear, and unchanged vibration parameters will suggest an adhesion dysfunction.

2. The method of claim 1 **characterized in that** the platelet function will be determined in more detail by adding different activators which will trigger different activation branches.

3. The method of one of the preceding claims **characterized in that** the activation branches will be blocked by the addition of respective inhibitors.

4. The method of claims 2 or 3 **characterized in that** activating and blocking individual receptors will allow identification of those that exhibit functional defects.

5. The method of one of the preceding claims **characterized in that** blocking or activating individual receptors or groups of receptors will be achieved through a specific antibody, through parts thereof, or through other specifically binding molecules such as lectins, fragments of nucleic acids or the like.

## Revendications

1. Procédé pour la détermination de paramètres de l'hémostase au moyen de la mesure de paramètres d'oscillation d'un résonateur avec une surface de mesure qui entre en contact avec un échantillon de fluide contenant des thrombocytes, la fréquence de résonnance étant évaluée à titre de paramètre d'oscillation, **caractérisé par** l'analyse des caractéristiques d'un paramètre d'oscillation sur la période en fonction de laquelle la fonction des thrombocytes est évaluée et il est discriminé entre le défaut d'adhésion et le défaut d'agrégation, ledit échantillon de fluide étant activé et, en cas d'une baisse essentiellement exponentielle des paramètres d'oscillation, les thrombocytes étant classés comme fonctionnels, en cas d'une baisse essentiellement linéaire des paramètres d'oscillation, la fonction des thrombocytes étant classée en tant que dysfonctionnement d'agrégation, mais toutefois avec une fonction d'adhésion fonctionnelle, et l'absence de changement dans les paramètres d'oscillation indiquant un dysfonctionnement d'adhésion.

2. Procédé selon la revendication 1, **caractérisé en ce que** la fonction des thrombocytes est précisée par l'ajout de différents activateurs qui déclenchent différentes branches d'activation.

3. Procédé selon l'une des revendications précédentes, **caractérisé en ce que** des branches d'activation.sont bloquées par l'ajout d'inhibiteurs correspondants.

4. Procédé selon l'une des revendications 2 ou 3, **caractérisé en ce que**, par l'activation et le blocage de récepteurs individuels, ceux présentant des défauts de fonctionnement sont identifiés.

5. Procédé selon l'une des revendications précédentes, **caractérisé en ce que** le blocage ou l'activation de groupes de récepteurs ou de récepteurs individuels est provoqué par un anticorps spécifique, par des composants de ce dernier ou par d'autres molécules de liaisons spécifiques, comme des lectines, des fragments d'acides nucléiques ou autres molécules similaires.
